# EUROPÄISCHE PATENTSCHRIFT

(11) **EP 0 386 503 B1**
(45) Veröffentlichungstag und Bekanntmachung des Hinweises auf die Patenterteilung: **31.08.1994**
(21) Anmeldenummer: 90103011.4
(22) Anmeldetag: 16.02.1990
(51) Int. Cl.: A61B 8/08

(54) **Ultraschallgerät zur Feststellung der Trächtigkeit von weiblichen Grosstieren**
Ultrasonic device for detecting pregnancy in female larger animals
Dispositif ultrasonique pour confirmer la gestation de grands animaux femelles

(30) Priorität: 23.02.1989 DE 3905567
(43) Veröffentlichungstag der Anmeldung: 12.09.1990
(73) Patentinhaber: Rheintechnik Weiland & Kaspar KG Maschinenfabrik., 56170 Bendorf (DE)
(72) Erfinder: Weiland, Werner, D-5413 Bendorf-Sayn (DE)
(74) Vertreter: Quermann, Helmut, Dipl.-Ing.

(56) Entgegenhaltungen:
- EP-A- 0 146 049
- EP-A- 0 173 837
- EP-A- 0 194 488
- US-A- 4 205 686

## Beschreibung

Die Erfindung betrifft ein Ultraschallgerät zur Feststellung der Trächtigkeit von weiblichen Großtieren, insbesondere Kühen und Pferden, mit einem Ultraschallkopf, der im Bereich einer mit einem viskosen Kontaktmittel versehenen Stirnfläche einen Ultraschallsender und einen Ultraschallempfänger aufweist und außen mit einem Griffansatz verbunden ist, sowie mit einem Gehäuse, in dem ein Signalgeber und eine Batterie zum Betreiben des Gerätes oder ein Stromanschluß für eine externe Stromquelle angeordnet sind, wobei Sender und Empfänger einerseits sowie Signalgeber und Batterie bzw. Stromanschluß andererseits durch ein Kabel verbunden sind.

Beim Messen mit Ultraschallgeräten ist es zur Erzielung aussagekräftiger Meßergebnisse unbedingt erforderlich, daß der Schallkopf mit seiner Stirnfläche dicht am zu schallenden Objekt anliegt, insbesondere, daß zwischen der Stirnfläche und dem Objekt kein Luftspalt verbleibt. In der Praxis wird deshalb auf die Stirnfläche des Schallkopfes ein Kontaktmittel aufgebracht, beispielsweise ein Kontaktgel oder -öl.

Bei weiblichen Großtieren, insbesondere Kühen und Pferden, bei denen mittels eines Ultraschallgerätes deren Trächtigkeit festgestellt werden soll, ergeben sich besondere Schwierigkeiten beim Messen deshalb, weil die Tiere nicht in Ruhe verharren. So ist es nicht nur erforderlich, den Schallkopf auf der Suche nach dem zu schallenden Objekt über die Haut bzw. das Fell des Tieres zu bewegen, mit der Gefahr, daß während des Bewegungsvorganges das Kontaktmittel zwischen dem Schallkopf und dem Tier entfernt wird, sondern es bringt die ständige Bewegung des Schallkopfes auch mit sich, daß eine sichere Anlage des Schallkopfes am Tier, das heißt ohne dazwischen befindlichen Luftspalt, kaum gewährleistet werden kann.

Aus der EP-A-0 173 873 ist ein Ultraschallgerät der eingangs genannten Art bekannt, bei der der Schallkopf manuell oder mittels einer Verlängerungsvorrichtung in den Mastdarm des Tieres eingeführt wird. Vor dem Einführen wird der Schallkopf mit Kontaktmittel benetzt. Beim manuellen Einführen liegt der Schallkopf in der Innenfläche einer Hand, die von einem ärmelartigen Einmalhandschuh umgeben ist. Beim Einführen des Schallkopfes mittels einer Verlängerungsvorrichtung wird anstelle des Handschuhes eine Überzugskappe aus Gummi oder Kunststoff verwendet. Der Handschuh bzw. die Überzugskappe ist aus hygienischen Gründen erforderlich, ferner um ein Abwischen des Kontaktmittels bei der Einführung zu verhindern. Eine derartige Überzugskappe hat aber den Nachteil, daß mit ihr, zusätzlich zum Koppelmedium, noch ein weiteres Medium zwischen das zu untersuchende Organ und den Schallkopf eingeschoben wird, wodurch das akustische Signal aufgrund der immer vorhandenen Impedanzsprünge an den Grenzschichten erheblich verschlechtert werden kann.

Es ist Aufgabe der vorliegenden Erfindung, den genannten Nachteil des Standes der Technik zu beheben und dabei gleichzeitig die Signalqualität zu verbessern.

Gelöst wird die Aufgabe bei einem Ultraschallgerät der eingangs genannten Art dadurch, daß mit dem Schallkopf im Bereich der Stirnfläche ein sich über die Stirnfläche hinaus erstreckendes und den Sender und Empfänger sowie das Kontaktmittel umgebendes elastisches Ringelement verbunden ist.

Die erfindungsgemäße Ausgestaltung des Schallkopfes stellt sicher, daß bei einem Bewegen des Schallkopfes auf der Haut bzw. dem Fell des Tieres das auf die dem Sender und Empfänger zugewandten Stirnfläche des Schallkopfes aufgebrachte Kontaktmittel innerhalb des vom elastischen Ringelement umgebenden Raumes verbleibt. Es versteht sich von allein, daß zweckmäßig das Kontaktmittel so dick auf den Schallkopf aufgebracht werden sollte, daß dessen freie Fläche mit dem freien umlaufenden Rand des elastischen Ringelementes abschließt. Beim Hin- und Herbewegen des Schallkopfes wird zwar aufgrund der Nachgiebigkeit des elastischen Ringelementes Kontaktmittel zwischen dem Ringelement und der Haut bzw. dem Fell des Tieres nach außen austreten können, es ist dabei aber immer sichergestellt, daß zwischen der Stirnfläche des Schallkopfes und der Haut bzw. dem Fell des Tieres ausschließlich Kontaktmittel verbleibt, somit sich dort kein Luftpolster befindet, das die Messung verfälschen würde. Aufgrund der besonderen Abdichtung des Schallkopfes zum zu schallenden Tier hin aufgrund des elastischen Ringelementes können nun größere Anlegeungenauigkeiten des Schallkopfes in Kauf genommen werden, was es möglich macht, am Schallkopf einen Griffansatz zu befestigen, womit der Schallkopf aus einer größeren Entfernung an das zu schallende Tier herangebracht werden kann und damit eine zusätzliche Sicherheit für die untersuchende Person gegeben ist. In der Praxis wird diese damit den Griffansatz zu seinem freien Ende ergreifen, auf die zweckmäßigerweise waagerecht ausgerichtete Stirnfläche des Schallkopfes das Kontaktmittel, beispielsweise das genannte Kontaktgel oder Kontaktöl aufbringen, bis auf Höhe des oberen Randes des elastischen Ringelementes, dann den Schallkopf an das zu schallende Tier heranführen, wobei das elastische Ringelement das Kontaktmittel nach außen abdichtet. Kippbewegungen des Schallkopfes führen dazu, daß das Ringelement auf einer Seite des Schallkopfes stärker zusammengedrückt wird als auf der gegenüberliegenden Seite des Schallkopfes, auch in diesem Zustand ist sichergestellt, daß das Ringelement ein Eindringen von Luft in den von diesem umgebenen Ringraum verhindert und damit die Voraussetzung für eine genaue Messung schafft, selbst dann, wenn der Schallkopf auf der Haut bzw. dem Fell des Tieres hin- und herbewegt werden muß. Die Erfindung ist dabei nicht darauf beschränkt, daß der Schallkopf und der Griffansatz separate Teile bilden, sie können genauso einteilig ausgebildet sein, beispielsweise aus einem Guß bestehen.

Eine bevorzugte Ausführungsform der Erfindung sieht vor, daß der Schallkopf als ein im Bereich der Stirnfläche geschlossenes und kreisförmigen Querschnitt aufweisendes Rohrstück ausgebildet ist, mit dessen Außenmantel benachbart der Stirnfläche das über die Stirnfläche sich hinaus erstreckende Ringelement verbunden ist. Die genannte Ausbildung des Rohrstückes ermöglicht eine baulich einfache Gestaltung des Schallkopfes mit einem in diesem Fall konkret kreisförmige Gestalt aufweisenden Ringelement. Das Ringelement selbst kann beispielsweise als flacher Gummiring ausgebildet sein. Die Verbindung des Ringelementes mit dem Schallkopf ist an sich beliebig, es wird jedoch für vorteilhaft erachtet, wenn das Ringelement mit dem Außenmantel des Rohrstückes verklebt ist und damit die beiden Teile dichtend miteinander verbunden sind.

Es ist jedoch nicht erforderlich, daß das Ringelement zwangsläufig mit dem Außenmantel des Rohrstückes verbunden sein muß, es besteht beispielsweise auch die Möglichkeit, in die Stirnfläche des Schallkopfes eine umlaufende Nut einzubringen, in die das Ringelement dichtend eingesetzt ist. Überdies sind zweckmäßig der Schallkopf und gegebenenfalls auch der Griffansatz mit einer Schutzhülle aus Gummi oder dgl. umgossen, die auch das elastische Ringelement teilweise umgeben kann.

Der Überstand des Ringelementes über die Stirnfläche des Schallkopfes bzw. die Schutzhülle des Schallkopfes bemißt sich nach den konkreten Erfordernissen und ist unter dem Aspekt zu sehen, daß eine Entfernung des Kontaktmittels vom Schallkopf verhindert werden soll. Ist beispielsweise ein Großtier zu untersuchen, das im zu schallenden Körperbereich ein dichtes Fell aufweist, wird zwangsläufig ein größerer Überstand des Ringelementes gefordert werden müssen als bei einem Tier, das nur ein dünnes Fell besitzt. In diesem Sinne kann durchaus ein Überstand bis zu 20 mm angezeigt sein, wobei übergroße Überstände ohnehin von untergeordnetem Einfluß sind, da beim Andrücken des Schallkopfes an das zu untersuchende Tier das elastische Ringelement nachgiebt und damit der Überstand, auch in Abhängigkeit von der Fellstärke, reduziert wird.

Damit das zum Schallkopf führende Kabel während des Meßvorganges nicht störend wirkt, ist vorgesehen, daß das Kabel durch den Griffansatz geführt ist. Vorteilhaft sollte darüberhinaus das dem Schallkopf abgewandte Ende des Griffansatzes ein Kupplungselement, insbesondere einen Stecker zum elektrischen Kuppeln eines ersten dem Gehäuse zugeordneten Kabelteils und eines zweiten, dem Schallkopf zugeordneten Kabelteils aufweist. Da es darüber hinaus erforderlich ist, daß der Schallkopf über eine größere Entfernung an das zu untersuchende Tier herangeführt werden muß, wird es für zweckmäßig erachtet, wenn das dem Schallkopf abgewandte Ende des Griffansatzes zwei Kupplungselemente, insbesondere Stecker zum mechanischen und elektrischen Verbinden mit einem Verlängerungsgriff bzw. einem im Verlängerungsgriff geführten Kabelteil aufweist.

In der Zeichnung der Figuren ist die Erfindung an einer Ausführungsform beispielsweise dargestellt, ohne auf diese Ausführungsform beschränkt zu sein.

Es zeigt:
- Figur 1: einen Längsschnitt durch das erfindungsgemäße Ultraschallgerät,
- Figur 2: eine Ansicht A gemäß Figur 1 auf den Schallkopf des erfindungsgemäßen Gerätes.

Das erfindungsgemäße Ultraschallgerät besteht im wesentlichen aus einem Schallkopf 1, einem mit diesem verbundenen ersten Griffansatz 2, einem mit letzterem verbundenen Verlängerungsgriff 3 sowie einem elektrisch mit dem Schallkopf 1 verbundenen Geräteteil 4.

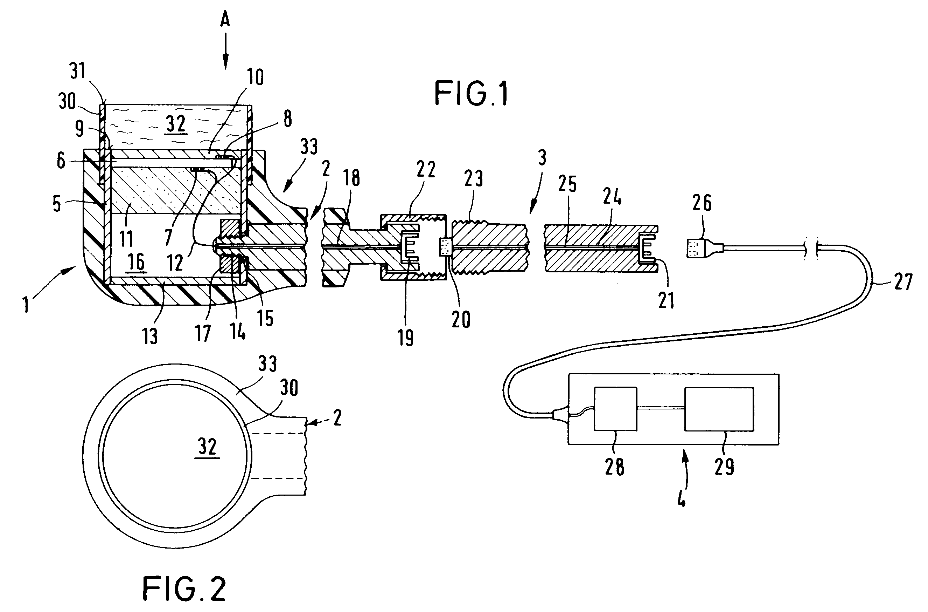

Im Detail besteht der Schallkopf 1 aus einem Rohrstück 5 kreisförmigen Querschnitts, an dessen, auf die Darstellung der Figur 1 bezogen, oberem Ende in Abstand von diesem Ende eine Piezooxidscheibe 6 angeordnet ist. Beidseitig dieser Scheibe sind die aktiven Elemente des Ultraschallsenders 7 bzw. des Ultraschallempfängers 8 angeordnet, zwischen der Piezooxidscheibe 6 und dem freien Ende des Rohrsrückes 5 befindet sich ein Koppelstück 10, dessen Querschnittsfläche dem lichten Querschnitt des Rohrstückes 5 entspricht. Die der Piezooxidscheibe 6 zugewandte Fläche des Koppelstückes 10 schließt bündig mit dem freien Ende des Rohrstückes 5 ab, das Koppelstück 10 ist dabei fest mit dem Rohrstück 5 verbunden. Auf der dem Koppelstück 10 abgewandten Seite der Piezooxidscheibe 6 ist eine Füllung 11 vorgesehen, durch die ein mit dem Ultraschallsender und Ultraschallempfänger 7 bzw. 8 verbundenes Elektrokabel 12 geführt ist. Das untere Ende des Rohrstückes 5 ist mit einer kreisförmigen Querschnitt aufweisenden Platte 13 verschlossen.

Der Griffansatz 2 durchsetzt mit einem verjüngten Gewindeabschnitt 14 eine Bohrung 15 im Mantel des Rohrstückes 5 und ragt in den zwischen der Füllung 11 und der Platte 13 gebildeten Hohlraum 16, eine Mutter 17 ist auf den Gewindeabschnitt 14 aufgeschraubt, womit der Griffansatz 2 fest mit dem Rohrstück 5 verbunden ist. Der Griffansatz 2 ist mit einer sich in dessen Längsrichtung erstreckenden Mittelbohrung 18 versehen, die das Elektrokabel 12 durchsetzt, das dem Gewindeabschnitt 14 abgewandte Ende weist ein negatives Steckerteil 19 auf, das mit dem Kabel 12 verbunden ist. Der Verlängerungsgriff 3 ist entsprechend dem Griffansatz 2 stabförmig ausgebildet, ein Ende des Verlängerungsgriffes 3 weist ein positives Steckerteil 20 auf, zum Einstecken in das negative Steckerteil des Griffansatzes 2, das andere Ende des Verlängerungsgriffes 3 ist mit einem negativen Steckerteil 21 versehen. In eingestecktem Zustand von Griffansatz 2 und Verlängerungsgriff 3 gelangt eine auf den Griffansatz 2 zwischen zwei Anschlägen axial verschiebbare Überwurfmutter 22 in Eingriff mit einem Gewindeabschnitt 23 des Verlängerungsgriffes 3. Aus zeichnerischen Gründen sind der Griffansatz 2 und der Verlängerungsgriff 3 voneinander getrennt dargestellt, beim Einsatz des erfindungsgemäßen Ultraschallgerätes stellen sie selbstverständlich eine bauliche Einheit dar.

Auch der Verlängerungsgriff 3 ist in dessen Längsrichtung mit einer Mittelbohrung 24 versehen, durch die ein Elektrokabel 25 geführt ist, welches die beiden Steckerteile 20 und 21 miteinander verbindet. Eine bauliche Einheit mit dem negativen Steckerteil 21 des Verlängerungsgriffes 3 bildet ein positives Steckerteil 26, das über ein weiteres Elektrokabel 27 mit dem Geräteteil 4 verbunden ist. In diesem befinden sich, wie in der Darstellung der Figur 1 schematisch angedeutet, als wesentliche Elemente ein elektrischer Signalgeber 28, beispielsweise ein akustisches Warngerät, ferner eine Batterie 29 zum Betreiben des Ultraschallgerätes. Die genannten Teile sind selbstverständlich elektrisch mit dem Elektrokabel 27 verbunden. Darüber hinaus können in dem Geräteteil 4 weitere für das Betreiben eines üblichen Ultraschallgerätes wesentliche Elemente untergebracht sein. In der Zeichnung der Figur 1 ist das Geräteteil 4 stark vereinfacht und verkleinert dargestellt. Darüber hinaus sind sowohl der Griffansatz 2 als auch der Verlängerungsgriff 3 unterbrochen dargestellt um zu verdeutlichen, daß je nach dem Anwendungsfall ein unterschiedlich langer Griffansatz 2 bzw. Verlängerungsgriff 3 zum Einsatz kommen kann.

Den beiden dargestellten Figuren ist darüber hinaus zu entnehmen, daß der Außenmantel des Rohrstückes 5 im Bereich seines freien Endes 9 mit einem elastischen Ringelement 30 versehen ist, welches sich über das freie Ende 9 hinaus erstreckt. Das Ringelement ist als flacher, schmiegsamer Gummiring ausgebildet, der im Bereich seines mit dem Rohrstück 5 in Anlage gelangenden Abschnittes mit diesem verklebt ist. Der Darstellung der Figur 1 ist zu entnehmen, daß der über das freie Ende 9 des Rohrstückes 5 hinausstehende Teil des Ringelementes 30 etwas größer ist als derjenige Teil, der mit dem Rohrstück 5 verklebt ist. Es liegt aber durchaus im Rahmen der Erfindung, den überstehenden Anteil des Ringelementes geringer auszubilden, was vom Anwendungsfall abhängig ist. In den zwischen dem freien Ende 9 des Rohrstückes 5 und dem freien Ende 31 des Ringelementes 30 vom Ringelement 30 umgebenen Raum ist ein Kontaktmittel 32, beispielsweise ein Kontaktgel eingebracht.

Der Darstellung der Figuren ist weiterhin zu entnehmen, daß der Schallkopf 1 und der Griffansatz 2 mit einem Schutzüberzug 33 aus Gummi versehen sind und dieser gummiummantelte Bereich etwa pfeifenform aufweist Schutzüberzug 33 umgibt bis auf Höhe des freien Endes 9 des Rohrstückes 5 das Ringelement 30.

Aufgrund der erfindungsgemäßen Ausgestaltung des Schallkopfes 1 mit dem elastischem Ringelement 30, welches das Kontaktmittel 32 umschließt und der Verbindung des Rohrstückes 5 des Schallkopfes 1 mit dem Griffansatz 2 und dem Verlängerungsgriff 3 besteht die Möglichkeit, auch solche weibliche Tiere auf Trächtigkeit hin zu untersuchen, denen sich der Untersuchende nicht ohne Gefährdung nähern kann. Der Griffansatz 2 und der Verlängerungsgriff 3 ermöglichen ihm, mit dem Schallkopf 1 größere Reichweiten zu überbrücken, das elastische Ringelement 30 verhindert, daß bei einer Bewegung des Schallkopfes 1 über das Fell bzw. die Haut des zu untersuchenden Tieres das Kontaktmittel abgerieben wird und sich zwischen dem Schallkopf 1 und dem Tier ein die Messung verfälschendes bzw. unmöglich machendes Luftpolster bildet.

## Patentansprüche

1. Ultraschallgerät zur Feststellung der Trächtigkeit von weiblichen Großtieren, insbesondere Kühen und Pferden,
mit einem Ultraschallkopf (1), der im Bereich einer mit einem viskosen Kontaktmittel (32) versehenen Stirnfläche einen Ultraschallsender (7) und einen Ultraschallempfänger (8) aufweist und außen mit einem Griffansatz (2, 3) verbunden ist,
sowie mit einem Gehäuse (4), in dem ein Signalgeber (28) und eine Batterie (29) zum Betreiben des Gerätes oder ein Stromanschluß für eine externe Stromquelle angeordnet sind, wobei Sender (7) und Empfänger (8) einerseits sowie Signalgeber (28) und Batterie (29) bzw. Stromanschluß andererseits durch ein Kabel (12) verbunden sind,
**dadurch gekennzeichnet**, daß mit dem Schallkopf (1) im Bereich der Stirnfläche (9) ein sich über die Stirnfläche (9) hinaus erstreckendes und den Sender (7) und Empfänger (8) sowie das Kontaktmittel (32) umgebendes elastisches Ringelement (30) verbunden ist.

2. Ultraschallgerät nach Anspruch 1, **dadurch gekennzeichnet**, daß der Schallkopf (1) als im Bereich der Stirnfläche (9) geschlossenes und kreisförmigen Querschnitt aufweisendes Rohrstück (5) ausgebildet ist und mit dessen Außenmantel benachbart der Stirnfläche (9) das über die Stirnfläche (9) sich hinaus erstreckende Ringelement (30) verbunden ist.

3. Ultraschallgerät nach Anspruch 1 oder 2, **dadurch gekennzeichnet**, daß dem Schallkopf (1) aus seiner der Stirnfläche (9) abgewandten Seite mit einem diesen verschliessenden Bodenteil (13) versehen ist.

4. Ultraschallgerät nach einem der Ansprüche 1 bis 3, **dadurch gekennzeichnet**, daß das Ringelement (30) als flacher Gummiring ausgebildet ist.

5. Ultraschallgerät nach einem der Ansprüche 1 bis 4, **dadurch gekennzeichnet**, daß das Ringelement (30) mit dem Außenmantel des Rohrstückes (5) verklebt ist.

6. Ultraschallgerät nach Anspruch 1, 3 oder 4, **dadurch gekennzeichnet**, daß in die Stirnfläche (9) des Schallkopfes (1) eine umlaufende Nut eingebracht ist, in die das Ringelement (30) eingesetzt ist.

7. Ultraschallgerät nach einem der Ansprüche 1 bis 6, **dadurch gekennzeichnet**, daß das Elektrokabel (12, 25, 27) durch den Griffansatz (2, 3) geführt ist.

8. Ultraschallgerät nach einem der Ansprüche 1 bis 7, **dadurch gekennzeichnet**, daß das dem Schallkopf (1) abgewandte Ende des Griffansatzes (2) ein Kupplungselement, insbesondere einen Stecker (19) zum elektrischen Kuppeln eines ersten, dem Gehäuse (4) zugeordneten Kabelteils (25, 27) und eines zweiten, dem Schallkopf (1) zugeordneten Kabelteils (12) aufweist.

9. Ultraschallgerät nach einem der Ansprüche 1 bis 8, **dadurch gekennzeichnet**, daß das dem Schallkopf (1) abgewandte Ende des Griffansatzes (2) Kupplungselemente (22, 19), insbesondere Stecker zum mechanischen und elektrischen Kuppeln mit einem Verlängerungsgriff (3) bzw. einem im Verlängerungsgriff (3) geführten Kabelteil (25, 27) aufweist.

10. Ultraschallgerät nach einem der Ansprüche 1 bis 9, **dadurch gekennzeichnet**, daß der Schallkopf (1) mit einer Schutzhülle (33), insbesondere einer aus Gummi bestehenden Schutzhülle (33) umgeben ist.

11. Ultraschallgerät nach Anspruch 10, **dadurch gekennzeichnet**, daß die Schutzhülle (33) das elastische Ringelement (30) teilweise umgibt.

12. Ultraschallgerät nach Anspruch 10 oder 11, **dadurch gekennzeichnet**, daß die Schutzhülle (33) den Griffansatz (2) umgibt.

13. Ultraschallgerät nach einem der Ansprüche 1 bis 12, **dadurch gekennzeichnet**, daß der Schallkopf (1) und der Griffansatz (2) einstückig ausgebildet sind.

## Claims

1. Ultrasonic device for determining the pregnancy of female large animals, especially cows and horses, having an ultrasonic head (1), which exhibits in the region of an end face provided with a viscous contact agent (32), an ultrasonic emitter (7) and an ultrasonic receiver (8) and is externally connected to a handle attachment (2, 3), and having a housing (4), in which a signal generator (28) and a battery (29) for operating the device or a power connection for an external power source are disposed, emitter (7) and receiver (8) on the one hand and signal generator (28) and battery (29) or respectively power connection on the other hand being connected by a cable (12), characterized in that an elastic ring element (30) extending beyond the end face (9) and surrounding the emitter (7) and receiver (8) and the contact agent (32) is connected to the acoustic head (1) in the region of the end face (9).

2. Ultrasonic device according to Claim 1, characterized in that the acoustic head (1) is designed as a pipe portion (5) which is closed in the region of the end face (9) and which exhibits a circular cross-section, and with the outer jacket of which, adjacent to the end face (9), the ring element (30) extending beyond the end face (9) is connected.

3. Ultrasonic device according to Claim 1 or 2, characterized in that the acoustic head (1) is provided, on its side remote from the end face (9), with a base portion (13) sealing the acoustic head.

4. Ultrasonic device according to one of Claims 1 to 3, characterized in that the ring element (30) is designed as a flat rubber ring.

5. Ultrasonic device according to one of Claims 1 to 4, characterized in that the ring element (30) is adhesively bonded to the outer jacket of the pipe portion (5).

6. Ultrasonic device according to Claim 1, 3 or 4, characterized in that a circulating groove is introduced into the end face (9) of the acoustic head (1), into which groove the ring element (30) is inserted.

7. Ultrasonic device according to one of Claims 1 to 6, characterized in that the electrical cable (12, 25, 27) is guided through the handle attachment (2, 3).

8. Ultrasonic device according to one of Claims 1 to 7, characterized in that that end of the handle attachment (2) which is remote from the acoustic head (1) exhibits a coupling element, in particular a plug (19), for the electrical coupling of a first cable portion (25, 27), associated with the housing (4), and a second cable portion (12), associated with the acoustic head (1).

9. Ultrasonic device according to one of Claims 1 to 8, characterized in that that end of the handle attachment (2) which is remote from the acoustic head (1) exhibits coupling elements (22, 19), in particular plugs, for the mechanical and electrical coupling with an extension handle (3) and respectively a cable portion (25, 27) guided in the extension handle (3).

10. Ultrasonic device according to one of Claims 1 to 9, characterized in that the acoustic head (1) is surrounded by a protective casing (33), in particular a protective casing (33) composed of rubber.

11. Ultrasonic device according to Claim 10, characterized in that the protective casing (33) partially surrounds the elastic ring element (30).

12. Ultrasonic device according to Claim 10 or 11, characterized in that the protective casing (33) surrounds the handle attachment (2).

13. Ultrasonic device according to one of Claims 1 to 12, characterized in that the acoustic head (1) and the handle attachment (2) are integrally designed.

## Revendications

1. Appareil à ultrasons servant à déterminer la gestation de gros animaux domestiques, en particulier des vaches et des juments, comprenant :
- une tête à ultrasons (1) qui, au niveau de sa face frontale recouverte d'un liquide de contact (32) visqueux, est équipée d'un émetteur (7) et d'un récepteur (8) d'ultrasons ainsi que d'un embout de manoeuvre (2, 3) dirigé vers l'extérieur,
- un boîtier (4) contenant un générateur de signaux (28) et une batterie (29) alimentant l'appareil , ou un raccordement à une source de courant extérieure, l'émetteur (7) et le récepteur (8) d'une part, le générateur de signaux (28) et la batterie (29) ou le raccordement d'autre part étant reliés par un câble (12),
caractérisé en ce que la tête (1), près de la face frontale (9) porte un élément annulaire élastique (30) s'élevant au-dessus de cette face et entourant l'émetteur (7), le récepteur (8) ainsi que le liquide de contact (32).

2. Appareil à ultrasons selon la revendication 1, caractérisé en ce que la tête (1) est constituée d'une pièce tubulaire (5) présentant une section transversale circulaire continue au niveau de la face frontale (9) et dont la paroi externe dans cette zone porte l'élément annulaire (30).

3. Appareil à ultrasons selon la revendication 1 ou 2, caractérisé en ce que la tête (1) du côté opposé à la face frontale (9) est obturée par un fond (13).

4. Appareil à ultrasons selon une des revendications 1 à 3, caractérisé en ce que l'élément annulaire (30) est un anneau de caoutchouc à paroi plane.

5. Appareil à ultrasons selon une des revendications 1 à 4, caractérisé en ce que l'élément annulaire (30) est collé sur la paroi externe de la pièce tubulaire (5).

6. Appareil à ultrasons selon la revendications 1, 3 ou 4, caractérisé en ce que la face frontale (9) de la tête (1) est munie d'une rainure périphérique dans laquelle est inséré l'élément annulaire (30).

7. Appareil à ultrasons selon une des revendication 1 à 6, caractérisé en ce que le câble électrique (12, 25, 27) est conduit à travers l'embout (2, 3).

8. Appareil à ultrasons selon une des revendications 1 à 7, caractérisé en ce que l'extrémité de l'embout (2) située à l'opposé de la tête (1) est équipée d'un élément d'accouplement, en particulier une prise (19) permettant d'assurer la liaison électrique entre un premier câble (25, 27) associé au boîtier (4) et un second câble (12) associé à la tête (1).

9. Appareil à ultrasons selon une des revendications 1 à 8, caractérisé en ce que l'extrémité de l'embout (2) située à l'opposé de la tête (1) porte des éléments d'accouplement (22, 19), en particulier une prise permettant l'accouplement mécanique et électrique respectivement avec un prolongateur (3) et un câble (25, 27) contenu dans ce prolongement.

10. Appareil selon une des revendications 1 à 9, caractérisé en ce que la tête (1) est entourée d'une enveloppe protectrice (33) faite en particulier de caoutchouc.

11. Appareil selon la revendication 10, caractérisé en ce que l'enveloppe protectrice (33) enveloppe partiellement l'élément annulaire élastique (30).

12. Appareil selon la revendication 10 ou 11, caractérisé en ce que l'enveloppe protectrice (33) enveloppe l'embout (2).

13. Appareil selon une des revendications 1 à 2, caractérisé en ce que la tête (1) et l'embout (2) ne forment qu'une seule pièce.
